Europäisches Patentamt

**European Patent Office**

Office européen des brevets

(19)

(11) Publication number: **0 310 410**

**A2**

(12)

# EUROPEAN PATENT APPLICATION

(21) Application number: **88309086.2**

(22) Date of filing: **30.09.88**

(51) Int. Cl.⁴: **A 61 K 35/56**

(30) Priority: **02.10.87 US 104199**

(43) Date of publication of application:
**05.04.89 Bulletin 89/14**

(84) Designated Contracting States:
**AT BE CH DE ES FR GB GR IT LI LU NL SE**

(71) Applicant: **Kawate, Tsuneyoshi**
**502 King of Prussia Road**
**Radnor Pennsylvania 19087 (US)**

**Ohnishi, Tsuyoshi**
**502 King of Prussia Road**
**Radnor Pennsylvania 19087 (US)**

(72) Inventor: **Ohnishi, Tsuyoshi**
**502 King of Prussia Road**
**Radnor Pennsylvania 19087 (US)**

(74) Representative: **Shaw, Laurence**
**George House George Road**
**Edgbaston Birmingham B15 1PG (GB)**

(54) Control of calcium activated potassium efflux from injured body cells.

(57) Charybdotoxin or the active fraction thereof, are useful in the treatment of conditions, eg. ischemia, hypoxia or contusion caused by calcium activated potassium efflux from cells. Pharmaceutical compositions are disclosed. The active fraction, identified as MR117, is claimed, together with the preparation thereof by enzyme digestion of charybdotoxin followed by isolation performed by chromatography.

EP 0 310 410 A2

**Description**

## CONTROL OF CALCIUM ACTIVATED POTASSIUM EFFLUX FROM INJURED BODY CELLS

The present invention relates to the control of calcium activated potassium efflux from injured body cells. It is known that most cells have potassium ion efflux channels which are activated by an increase of cellular calcium concentration (Gardos, Biochim. Biophys. Acta 30:653-654, 1958). Therefore, when an increase of cellular calcium ions is caused in organ cells or in red blood cells by conditions such as ischemia, hypoxia or by physical-chemical damage to their membranes, cells lose potassium ions, which causes imbalance of cellular electrolytes and water, and ultimately kills the cells.

The invention is based on the realisation that charybdotoxin (a scorpion venom) and an isolated active fraction thereof are useful in the control of calcium activated potassium efflux.

According to the invention in one aspect, there is provided a pharmaceutical composition for use in inhibiting calcium activated potassium efflux and the like, comprising an effective amount of charybdotoxin or an active fraction thereof, in association with a pharmaceutical carrier or diluent.

Preferably the active fraction has been obtained by enzyme digestion of charybdotoxin. Most preferably the active fraction is the material designated hereinafter as MR117

The composition is preferably adapted for oral, subcutaneous or intravenous administration. The composition is preferably adapted for oral administration at the rate of 0.2 m/kg body weight/day, or for subcutaneous administration at the rate of 0.15 mg/kg body weight/day or for intravenous administration at the rate of 0.1 mg/kg body weight/day. Preferably the composition is in unit dosage form and for preventative or therapeutic treatment of conditions such as ischemia, hypoxia, contusion or the like, as explained hereinafter.

In another aspect, the invention provides an active fraction of charybdotoxin, having a molecular weight of about 3600 and designated herein as MR117.

The invention also provides a method of making an active fraction of charybdotoxin, characterised by subjecting charybdotoxin to enzyme digestion, and then subjecting the product to chromatography and isolating the active fraction.

Preferably the enzyme digestion is carried out using pepsin, V-8 protease or pyroglutamate aminopeptidase, and the chromatography is carried out on a HPLC column, and elution is carried out using sodium sulphate and a phosphate buffer.

In order that the invention may be well understood, it will now be described by way of illustration only with reference to the following examples and the accompanying drawings, which relate to information in the examples.

EXAMPLE I

Preparation

a) Preparation of charybdotoxin (MR-103) from scorpion venom.

A modified version of the method of Smith et al (J Biol Chem 261:14607-14613, 1986) was used. A 200mg sample of L quinquestriatus venom was dissolved in 35ml of buffer A (40mM NaCl, 10 mM Na-borate, 10 mM $Na_2CO_3$, pH 9.0), and the undissolved mucoid material was removed by centrifugation at 5000 x g for 10 minutes. The supernatant was saved and the pellet extracted twice in 10ml of buffer A. The combined supernatant was loaded onto a 5ml column of Sp-Sephadex equilibrated with buffer A. The column was washed with 35ml of buffer A, until the $A_{280}$ of the eluate fell to below 0.08. A linear gradient (80ml total volume) of buffer A to buffer B (20mM NaC1, 10 mM $Na_2CO_3$, 10 mM $Na_2HPO_4$, pH 12.0) was then applied to the column, and the protein concentration and pH of the eluent was followed. The peak of MR-103 activity was found to elute at pH 10.8-11.0.

Active fractions from the Sp-Sephadex column were neutralised by addition of acetic acid, and were further fractionated by reversed-phase HPLC, using a 150A pore C8 column. Sample (about 200 ug protein) was applied to the column, which was then washed with 5ml of buffer C (41mM acetic acid, 9mM ammonium acetate, pH 3.9). A linear gradient (l5ml total volume) of buffer C to buffer D (50mM acetic acid in 50% methanol) was run at 1ml/min, and $A_{280}$ was monitored. The major peak of MR-103 was eluted at 25-30% methanol. Purified MR-103 was freeze-dried and stored at -20°C.

b) Preparation of subfragment MR-117 from MR-103:

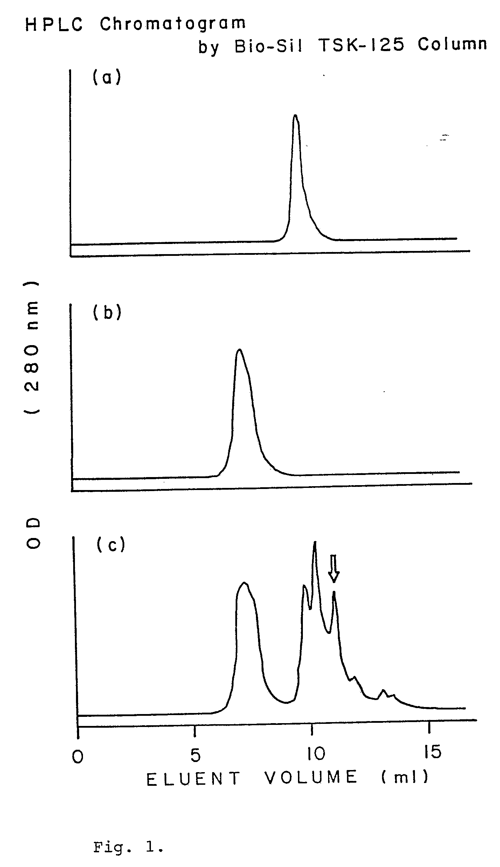

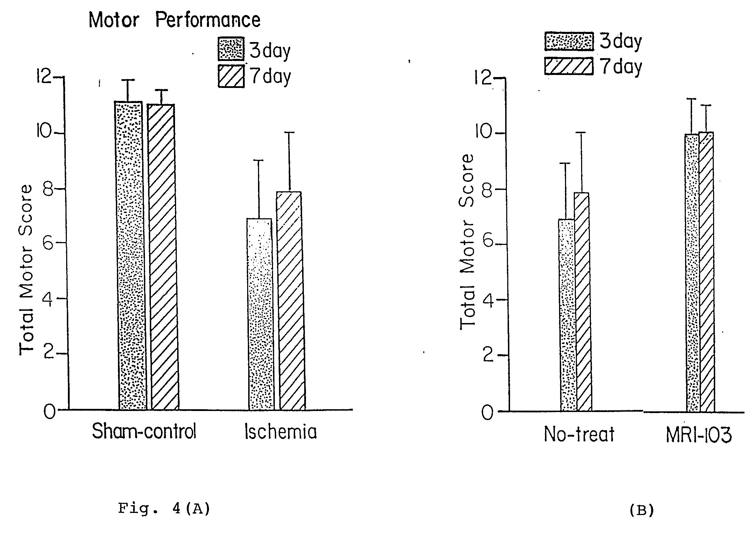

A solution containing 0.5mg/ml of MR-103, 0.5mg/ml of pepsin and 10mM glycine buffer (pH 2.5) was incubated at 30°C for 12 hours. Then the solution was applied to a Bio-Sil TSK-125 (7.5x300mm) HPLC column. Then it was eluted by a solution containing 50mM sodium sulphate and 20mM phosphate buffer (pH3.8) at the velocity of 1ml/min. The accompanying Figure 4 shows elution patterns of (A) MR-103, (B) pepsin and (C) the mixture. The first peak of the chromatogram of MR-l03-pepsin mixture (Fig 1 (C)) was pepsin itself. Other three peaks were digested subfragments of MR-103. Only the third peak, which had a molecular weight of 3,600, was found to have inhibitory activity against calcium-activated potassium efflux. This compound is named as MR-117 for an identification purpose.

Toxicity test:

Acute toxicity (LD$_{50}$) was determined using mice, to which drugs were injected i.p. The LD$_{50}$ of MR-103 and MR-117 were 0.79mg/kg and 0.92g/kg, respectively.

EXAMPLE II

Effect of drugs on liver ischemia

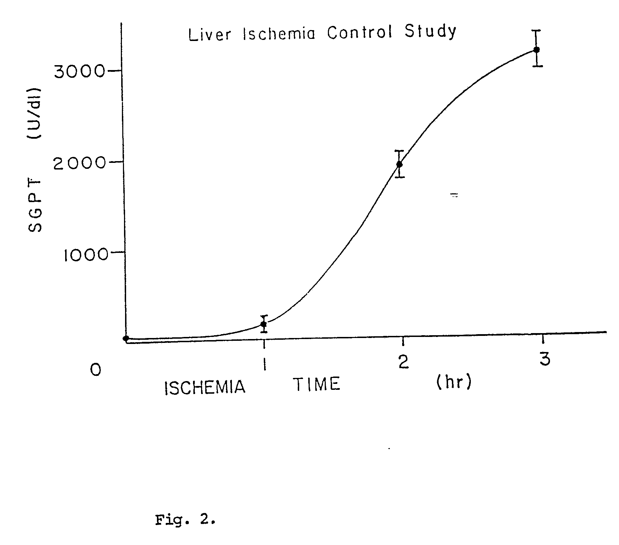

Rats weighing 150 to 175 grams were used. Under enflurane anesthesia, a microvascular clamp was placed to occlude the portal vein as well as the hepatic artery, which supplied blood to the left lateral and median lobes of the liver. The rats were subjected to ischemic conditions ranging from 1 to 3 hours, after which reperfusion of the liver is allowed by removing the clamp. Twenty four hours later, blood samples were drawn for the SGPT (serum glutamate-pyruvate transaminase) assay. As shown in the results of Figure 2, after 2 or 3 hours ischemia the SGPT level steeply increased (to levels of 2,000 to 3,000 U/dl) from the control level which is about 100 U/dl. The drug was tested using 2 hours of ischemia. As shown in Table 1 below, MR-103 suppressed the increase of SGPT levels by about 60%.

EXAMPLE III

Effects of drugs on brain ischemia

(a) Surgical procedures:

Adults male Sprague-Dawley rats weighing 250-300 g were used. Anesthesia was induced and maintained with halothane (1,0-2.5%) via a closely fitted facial mask. Bilateral common carotid arteries (CCA) were exposed gently and the right CCA was ligated in two placed by 4-0 silk suture. To expose the right middle cerebral artery (MCA) the temporal muscles were cut and retracted, then, a small temporal craniotomy (2x2mm) was performed with a microdrill. Under a microscope, the dura mater was opened with a 26 gauge needle, and the MCA was ligated by 10-0 suture with a square knot. Immediately, the left CCA was occluded by a microaneurysmal clip. After the operation, the animal was returned to its cage. One hour later, the left CCA clip was released in awake condition. The sham-operated control was produced in a similar procedure, except for the ligations and clipping of vessels. The operation was usually finished within 30 minutes.

b) Water content and ionic analysis

After fixed time intervals (1, 3 and 7 days), the animal was sacrificed and the excised cerebral hemisphere was placed in a preweighed crucible and measured and wet weight (WW) with a chemical balance (Mettler A E 100). The sample was then dried in an oven at 105°C until it reached a constant weight, which is defined as a dry weight (DW). The water content (% wet weight) was calculated (WW - DW)/WW.

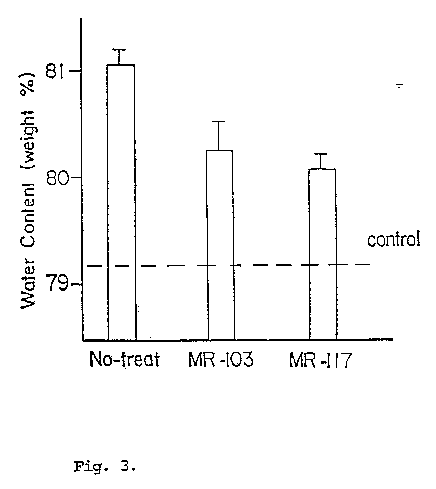

The water content of the right hemisphere (ischemia side) was increased, showing that the brain edema developed. 0.14mg/kg of either MR-103 or MR-117 was administered i.v. thirty minutes prior to the ischemia insult, the increase of water content was much reduced as shown in the results of Figure 3.

(c) Motor performance test:

The disturbance of motor functions was evaluated by the total score obtained from the inclined plate test, balance beam test and prehensile test. These motor performance tests were designed to quantify the motor weakness in the global ischemia rats. The original method was modified to suit to the rat focal ischemia model employed in this study, whose motor deficits may be less severe than that of global ischemia (4-vessel occlusion, 20 min). Two trials were given to the animal, and the better score was chosen as its data. To lessen the factor of fatigue, the trial was carried out at intervals of a few minutes. In the inclined plate test, a 60 x 30 cm board covered with a thin rubber pad was used as a plate and was fixed at a designated angle (55°). The animal was placed at the highest position of the plate and the time which it could stay on the plate was measured until 30 seconds. In the balance beam test, a wooden rod, 70 cm long and 3.2 cm diameter, was positioned horizontally 60 cm above a thick sponge pad and the animal was left at the centre of rod. The time that the rat spent on the rod was recorded until 30 second. In the prehensile test, a Nylon rope, 70 cm long and 4mm in diameter, was stretched horizontally 60 cm above a thick sponge pad and the rat's forepaws were placed on the rope and then released. The time that the rat spent hanging on the rope was measured until 30 seconds. The scoring methods in these three tests were identical and defined as follows; 0:0 second; 1;1-10 seconds; 2:11-20 seconds; 3:21-30 seconds; 4:above 30 seconds. Therefore, the total motor score ranged from 0 to 12 points.

Figure 4 shows in block diagram form the motor function as determined by three tests (complete paralysis is indicated by motor score 0, and normal condition as 12). By focal ischemia, 3 days later, the animal lost about 50% of motor ability as indicated by the motor score of 7 in non-treated animals. In 7 days, animals recovered to approximately the score of 8. With 0.14mg/kg of MR-103 given before ischemia, the motor score obtained 7 days after the operation was about 11 indicating that animals were almost completely recovered. The results in the case of MR117 are given in Table 2.

(d) Passive avoidance test:

The apparatus comprised an illuminated large compartment (40x40cm) and a dark small compartment (10x10cm) interconnected by an opening (6x6cm). Grids made of metal conductors were placed at the bottom of the small compartment. The rat was placed on a fixed location in the large compartment and was allowed to explore the apparatus for 180 seconds. The time spent in the small compartment was recorded with a stop watch. Then, when the rat was in the small compartment, the opening was closed with a transparent shutter and the rat received a weak electric shock for 30 seconds (to a degree that the rat felt a slight pain). By this method, the rat had an experience of fear (learning) and passive avoidance reaction was established. The retention test was undertaken in the same manner as the first exploration 24 hours after the initial test.

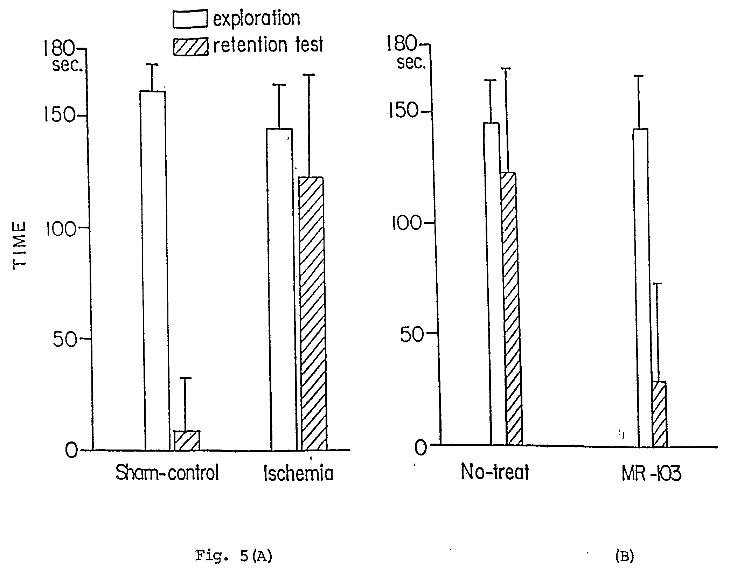

After receiving the electric shock, normal rats remembered the pain and never went back to the dark room again (retention time was 0). However, in ischemia rats, their memory was disturbed; they went back into the dark room. The results of Figure 5 shows that ischemic rats spent approximately 120 seconds in the dark room (as shown by shadowed column of retention time). When the drugs were administered before the ischemia, animals maintained better memory as shown in the results of Figure 5 The retention time was significantly decreased (to 40 to 50 seconds).

EXAMPLE IV

Spinal Cord Injury

In spinal cord injury, the primary injury is accompanied by immediate loss of nerve action potential and rapid ion movement. This is followed by secondary events which results in cell death in a matter of hours.

a) Experimental procedure:

A method developed by Wrathall's group (Wrathall et al, Exper Neurology 88:108-122, 1985; Noble L J and Wrathall J R, Exper Neurology 95:530-533, 1987) was used. Animals were anesthetised with nembutal (50mg/kg). Laminectomy was performed on the T-11 segment of the spinal cord. The skin wound was temporarily closed and the animal recovered from the effect of laminectomy. Several hours later, the animal was anesthetised with 1.5% halothane (air as carrier). The T-11 segment was again exposed and an impounder (made of plastic with a tip diameter of 2.4mm) was placed on the exposed dura. Then, a 10 gram weight was dropped from the height of 5cm onto the impounder. The impounder remained on the surface of the spinal cord for a few seconds, after which it was removed. The skin wound was sutured and closed. The animal was returned to the cage after regaining consciousness. The bladders were compressed twice daily to induce urination.

b) Assessment of motor recovery:

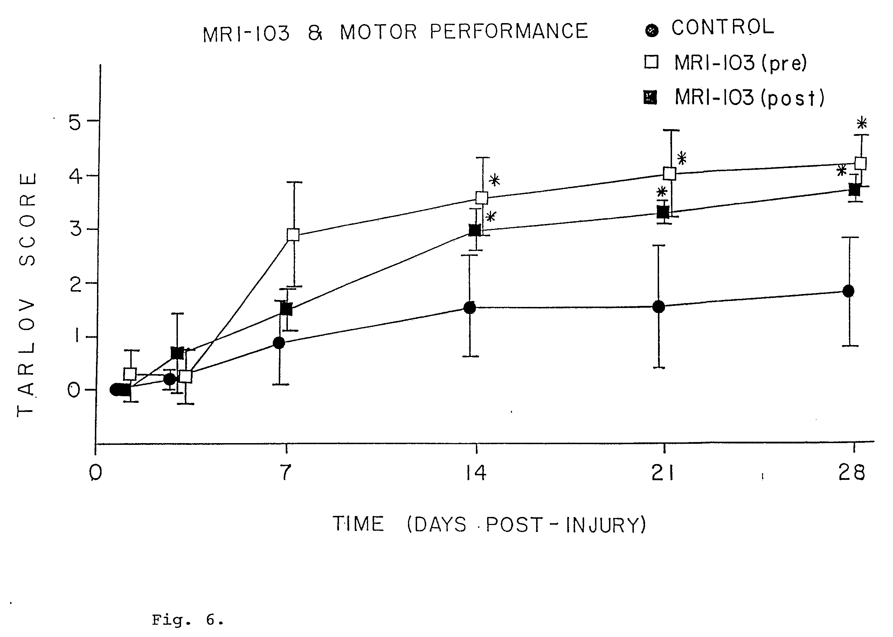

After contusion, animals were observed for four weeks during which time the recovery was quantified by rating the hind-limb walking ability according to the method originally used by Tarlov (Tarlov, I M Arch Neurol psychiatry 71:588-597, 1954) and modified by Wrathall et al. (Exper. Neurology 88:108-127, 1985). This is basically a scoring system for hind limb mobility ranging from 0 to 5. Zero indicates complete paralysis while a score of 5 indicates full recovery. As shown in Figure 6, measurements were taken from a group of rats receiving no treatment (control) and a group receiving drug treatment after injury. The drug MR-103 was administered i.v. at the rate of 0.14mg/kg body weight. Pretreatment was more effective than post treatment.

EXAMPLE V

Sickle cell anaemia

Irreversibly sickled cells (ISC, which are irreversibly denatured sickle cells) have been produced by exposing sickle red blood cells to a repeated sickling-unsickling cycling in vitro and this can be used to determine the efficacy of drugs in protecting cell membranes (Ohnishi, Br J Haematol 55:665-671, 1983; Ohnishi et al Biochim Biophys Acta 886:119-129, 1986). When sickle cells were exposed to a sickling-unsickling cycling for 3 hours, about half of the cells were denatured as revealed by the formation of heavy, dehydrated cells in density gradient centrifugation. These heavy cells were collected, the amount measured, and the percentage of production calculated (Table 3). As shown in the Table, when 20 nM of MR-103 was added, the formation of irreversibly denatured cells was inhibited by almost 50%, and at 60nM it was almost completely inhibited. MR-117 was also effective: at 60nM it inhibited the formation of denatured cells by more than 50%.

Table 1

Protective effect of MR-103 against liver ischemia of rats.

SGPT values were measured 24 hours after 2 hours global ischemia.

| Dose | SGPT (U/dl) |
|---|---|
| No drug | 1,927.1 |
| MR-103 (0.05 mg/kg) | 756.3 |

Table 2

Protective effect of MR-117 against the loss of motor performance caused by brain focal ischemia.

Data was taken 3 days after ischemia.

| Condition | Motor Score |
|---|---|
| No drug | $6.93 \pm 0.54$ |
| MR-117 (0.14 mg/kg) | $9.50 \pm 0.56$ |
| Sham control | $11.13 \pm 0.28$ |

Table 3

Protective effect of MR-103 against the formation of irreversibly sickled cells caused by sickling-unsickling cycling (numbers indicate the percentages)

| | Drug during cycling | | | No cycling | |
|---|---|---|---|---|---|
| | 0 | 20 | 60um | 120 | 0(nM) |
| Reversibly sickling cells | 10.3 | 47.2 | 95.6 | 100 | 100 |
| Irreversibly sickled cells | 89.7 | 52.8 | 4.4 | 0 | 0 |

Protective effect of MR-117 against the formation of irreversibly sickled cells caused by sickling-unsickling cycling (numbers indicate the percentages)

| | Drug during cycling | | | No cycling | |
|---|---|---|---|---|---|
| | 0 | 20 | 60 | 120 | 0(nM) |
| Reversibly sickling cells | 64.1 | 85.2 | 89.7 | 100 | 100 |
| Irreversibly sickled cells | 35.9 | 14.8 | 10.3 | 0 | 0 |

**Claims**

1. A pharmaceutical composition for use in inhibiting calcium activated potassium efflux and the like, comprising an effective amount of charybdotoxin or an active fraction thereof, in association with a pharmaceutical carrier or diluent.

2. A composition according to Claim 1 characterised in that the active fraction has been obtained by enzyme digestion of charybdotoxin.

3. A composition according to Claim 2 characterised in that the active fraction is the material designated in the description as MR 117.

4. A composition according to any of Claims 1 to 3 characterised in that the composition is adapted for oral, subcutaneous or intravenous administration.

5. A composition according to Claim 4 characterised in that the composition is adapted for oral administration at the rate of 0.2 m/kg body weight/day, or for subcutaneous administration at the rate of 0.15 mg/kg body weight/day or for intravenous administration at the rate of 0.1 mg/kg body weight/day.

6. A composition according to any preceding Claim characterised in that the composition is in a unit from for use in ischemia, hypoxia and contusion.

7. An active fraction of charybdotoxin, having a molecular weight of about 3600 and designated herein as MR117.

8. A method of making an active fraction of charybdotoxin, characterised by subjecting charybdotoxin to enzyme digestion, and then subjecting the product to chromatography and isolating the active fraction.

9. A method according to Claim 8 characterised in that the enzyme digestion is carried out using pepsin, V-8 protease or pyroglutamate aminopeptidasae.

10. A method according to Claim 8 or 9 characterised in that the chromatography is carried out on a HPLC column, and elution is carried out using sodium sulphate and a phosphate buffer.

HPLC Chromatogram
    by Bio-Sil TSK-125 Column

Fig. 1.

Fig. 2.

Fig. 3.

Fig. 4(A)

(B)

Fig. 5(A)                    (B)

EP 0 310 410 A2

Fig. 6.

MRI-IO3 & MOTOR PERFORMANCE

TARLOV SCORE

TIME (DAYS POST-INJURY)

● CONTROL
□ MRI-IO3 (pre)
▨ MRI-IO3 (post)